# EUROPEAN PATENT SPECIFICATION

(11) **EP 0 327 356 B1**
(45) Date of publication and mention of the grant of the patent: **22.03.1995**
(21) Application number: 89301001.7
(22) Date of filing: 02.02.1989
(51) Int. Cl.: C07D 301/10, B01J 23/66, B01J 23/50

(54) **High efficiency silver catalysts for the production of ethylene oxide via ethylene oxidation**
Hochwirksame Silberkatalysatoren zur Herstellung von Ethylenoxid durch Ethylenoxidation
Catalyseurs à argent à haute efficacité, pour l'oxydation de l'éthylène en oxyde d'éthylène

(30) Priority: 03.02.1988 CN 88100400
(43) Date of publication of application: 09.08.1989
(73) Proprietor: CHINA PETROCHEMICAL CORPORATION, Beijing (CN); RESEARCH INSTITUTE OF BEIJING YANSHAN PETROCHEMICAL CORPORATION, Yanshan District Beijing (CN)
(72) Inventor: Jin, Jiquan Research Institute of Beijing Yanshan, Yanshan District Beijing (CN); Jin, Guoquan Research Institute of Beijing Yanshan, Yanshan District Beijing (CN); Xu, Yong Research Institute of Beijing Yanshan, Yanshan District Beijing (CN); Shang, Liandi Tianjin Chemical Research Institute, Tianjin (CN); Luo, Guochun Tianjin Chemical Research Institute, Tianjin (CN)
(74) Representative: Knowles, Audrey Elizabeth

(56) References cited:
- EP-A- 0 000 460
- EP-A- 0 058 419
- EP-A- 0 244 895
- EP-A- 0 247 414
- GB-A- 1 465 523
- US-A- 3 172 893

## Description

This invention relates to a process for preparing silver-containing catalysts and their carriers for the production of ethylene oxide via ethylene oxidation.

While ethylene oxide is produced by ethylene oxidation, carbon dioxide is also formed by the side reaction. The reaction heat of the side reaction is twenty times that of the main reaction and the ethylene oxide produced in the main reaction will further be oxidised into carbon dioxide if it is not removed in time.

In order to increase the activity of the catalysts, sufficient specific surface area of silver particles must be afforded. Therefore, the catalyst carriers are required to have enough specific surface area. However, oversized specific surface area will make the transfer of the reaction heat difficult, aggravate the side reaction and decrease the selectivity of the catalysts.

In order to offer the catalysts a high selectivelty, an ideal pore structure which matches the surface of the catalyst is required so that suitable conditions for heat and mass transfer can be attained and the side reaction can be suppressed.

Since the reaction takes place under nearly diffusion controlling conditions, searching for carriers with an optimum matching between pore structure and specific surface area has become an important subject in developing silver catalysts with a high selectivity.

US-A-4,379,134 (& EP-A-0,058,419) discloses a process for the production of alpha-alumina bodies that are useful as catalyst supports having a specific surface area of less than one square meter per gram with at least 85 percent of the pore volume represented by pores having a diameter of from 1 to 20 micrometres (microns) (radius 0.5 to 10 micrometres), and not more than 10 percent of the pore volume represented by pores having a diameter from 20 to 100 micrometres (radius 10 to 50 micrometres) formed by incorporating carbonaceous material in the production of the alumina carriers.

GB-A-1,465,523 discloses a method for producing shaped bodies of alumina for use in catalytic applications having a wide range of specific surface area from 0.1 to 60 m²/gm and a pore volume greater than 0.1 ml/gram represented by pores larger than 0.1 micrometres, exclusive of pores produced by burnout material.

US-A-3,172,893 discloses a catalyst for the production of ethylene oxide via ethylene oxidation comprising silver coated pellets formed by bonding alumina particles of uniform relatively large diameter in the range 400-600 micrometres and pores having a diameter of from 80-200 micrometres (radius 40-100 micrometres).

EP-A-0,244,895 discloses a silver catalyst for the oxidation of ethylene to ethylene oxide comprising alumina particles enriched with an alkali metal oxide or hydroxide promoter and a fluoride anion each present in an amount between 10 and 1000 parts by weight per million parts by weight of the total catalyst.

EP-A-0,247,414 discloses a silver deposited catalyst for producing ethylene oxide by ethylene oxidation having an alumina carrier with a surface area of 0.6 to 2 m²/gm, a silica content of 0.5 to 12 percent by weight, and a sodium content of 0.08 to 2 percent by weight.

The purpose of the present invention is to avoid the disadvantages arising from oversized or undersized pore structure and specific surface area and inadequate pore size distribution in the prior art, and to provide alumina carriers having a better matching specific surface area and pore structure, and to greatly increase the selectivity of the silver catalysts in commercial applications.

The purpose of the invention is achieved by the process for producing a silver catalyst defined in Claim 1.

According to the requirement of the pore formation, trihydrated α-alumina and boehmite alumina having different particle sizes are used as starting materials with carbonaceous material having matching particle sizes to provide the alumina carriers with the desired pore structure. Advantageously, the alumina carriers have a pore structure with a specific surface area of 0.8-1.3 m²/gm and a pore volume of 0.5-0.7 ml/gm.

Unlike the process disclosed in US-A-4,379,134 which uses expensive boehmite alumina, the present invention uses a mixture of boehmite alumina and cheaper α-alumina, preferably commercially available trihydrated α-alumina having a low sodium content.

The carbonaceous materials are used to make the large pores and include petroleum coke, carbon powder, graphite, polyethylene, rosin and mixtures thereof. The carbonaceous materials are carbonised and oxidised and become gas to escape during calcination. In this way, large pores are formed in the carriers which benefit the diffusion and heat transfer of the reaction gases in the catalysts.

Although US-A-4,379,134 uses carbonaceous materials to make large pores with reference to US-A-3,726,811 and US-A-3,119,660, the fraction of the large pores having a diameter greater than 20 micrometres (radius 10 micrometres) does not exceed 10 percent of the total pore volume. The present invention on the other hand shows that, if the fraction of large pores having a radius greater than 30 micrometres (diameter 60 micrometres) is from 10 to 25 percent of the total pore volume, the selectivity of the catalyst will increase significantly.

If excess carbonaceous materials are added, the strength of the alumina carriers will decrease and, according to a preferred feature of the invention, the added carbonaceous material ranges from 20 to 30 percent of the weight of the alumina.

Fluxing agents can decrease the calcination temperature and make the alumina carriers have enough crush strength. The preferred fluxing agents used in the invention consist of magnesium nitrate, magnesium oxide and feldspar. The amount of the fluxing agent preferably ranges from 1.5 to 7 percent of the alumina by weight. The crush strength of the alumina carriers is preferably greater than 5 kg/particle based on a particle having an outer diameter of 6.5mm, an inner diameter of 2.5mm and a length of 6.5mm.

Binders make crystal alumina disperse and bind during mixing to form an extrudable paste. The preferred binders used in the invention include nitric acid, alumina gel, propanoic acid, acetic acid and formic acid. The amount of added nitric acid or alumina gel preferably ranges from 25 to 60 percent of the alumina by weight.

Fluorides make alumina easy to transform into crystals and the alumina is converted completely into α-alumina crystals during the calcination which benefits the elimination of unnecessary micropores. The preferred fluorides used in the invention consist of ammonium fluoride, hydrogen fluoride and aluminum fluoride. The amount of the added fluoride anions preferably ranges from 0.5 to 5.0 percent of the alumina by weight.

After being extruded and shaped, the paste is preferably dried to a water content of less than 30 percent. The carrier bodies can take the shape of rings, balls, cylinders or perforated cylinders. The carrier bodies are preferably dried at 80°C to 120°C for a period of 1 to 24 hours which is controlled according to the water content.

After being dried, the carrier bodies are preferably heated to a temperature from 1450°C to 1550°C and kept at the temperature for about 2 to 6 hours so that all the alumina is converted to α-alumina. Meanwhile, the fluoride anions can eliminate micropores to make the pores having the radius of less than 30 micrometres range mainly from 0.5 micrometres to 5 micrometres, and the pores having the radius of larger than 30 micrometres range from 25 to 10 percent of the total volume.

Silver catalysts are prepared from the alumina bodies by impregnating them with solutions of silver compounds that are preferably sufficient to allow the silver to be supported on the carrier in an amount from 1-25% of the weight of the catalyst. The impregnated carriers are then separated from the solution and the silver compound is reduced to silver. When or before or after the silver compound is supported on the carriers, in addition to the silver compound, one or more promoters chosen from alkali metals such as potassium, rubidium and caesium or alkaline earth metals such as barium is/are supported on the carriers.

According to a preferred method, silver oxalate is precipitated by mixing aqueous solutions of silver nitrate and ammonium oxalate. The precipitate is then filtered and washed to eliminate nitrate anions. The silver oxalate is then dissolved in an aqueous solution of ethyldiamine and diethanolamine and promoters are added to form an impregnation solution. The alumina carrier is impregnated with the impregnation solution and drained and heated in an air flow at a temperature from 550°C to 600°C for a duration to produce a silver catalyst containing 5 to 20 percent of silver by weight. Silver nitrate can be replaced by silver oxide in the above method. Alternatively, silver oxalate can directly form a complex with amines without filtration and then the alumina carriers are impregnated with the complex solution.

Promoters of alkali metals such as potassium, rubidium and caesium are usually doped onto the catalyst in amounts (calculated on metal) of from 20, more preferably 40, to 1000 parts per million parts of the catalyst by weight, and promoters of alkaline earth metals such as barium are doped in amounts (calculated on metal) of less than 1000 parts per million parts of the catalyst by weight.

The alkali metals deposited on the alumina carriers can be washed with absolute alcohol or absolute methanol. The concentration of the alkali metals can be controlled in a certain range because some of the alkali metals are cleaned away.

All kinds of the silver catalysts prepared by the process of the invention are evaluated in a single tube reactor or micro reactor. The single tube reactor has a diameter of 21mm, a 7.07m catalyst bed height, and an effective volume of 2.5 litres.

| Constitution of the reacting Gas: | |
|---|---|
| C₂H₄ | 12-25% (mole concentrations) |
| O₂ | 6.5%-7.5% |
| CO₂ | 6-10̸% |
| N₂ | rest |
| Dichloroethane | 0̸.0̸5-1.0̸5 ppm |
| Space Velocity | 70̸0̸0̸/hour |
| Temperature | 220̸-245°C |
| Pressure | 21Kg/cm² |
| Ethylene oxide in the Effluent | 1.3-1.6% |
| Time-Space Yield | 170̸-260̸g Ethylene Oxide/l Cat. h |

The constitution of the inlet and exit gases of the reactor are measured continuously when the conditions described above are reached. The selectivity of the catalyst is calculated by the following equation after correcting the data for volume thrinkage. 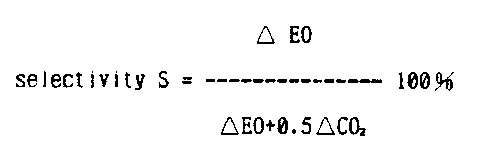
Wherein , ΔEO is the concentration difference of the ethylene oxide between exit and inlet gases.

Once the termperature remains unchanged (between 220̸° and 245°C ) with a constant conversion and the maximum selectity , the continous measurement is carried out again and the selectivity is calculated by averaging the data of more than 30̸ groups.

Compared with the prior arts , the present invention has the following advantage : the silver catalysts prepared by the process of the invention are particularly suitable for ethylene oxidization to produce ethylene oxide and has a selectivity of from 83 to 84 percent in the conditions disclosed above in industrial facilities.

The invention will be further discribed in detail in the following examples:

### Example 1

77 kilograms of trihydrated α-alumina of less than 50̸ mesh with low sodium content , 23 kilograms of boehmite alumina of less than 20̸0̸ mesh , 1.7 kilograms of ammonium fluoride , 20̸ kilograms of petroleum coke of 30̸-20̸0̸ mesh, 2 kilograms of magnesium nitrate were uniformly mixed in a mixer. 20̸ kilograms of said mixture were placed into a kneading machine , to which 2.7 liters of dilute nitric acid (acid:water=1:3)and an adequate amount of water were added. The mixture was kneaded to form an extrudable blend. Said extrudable blend was then extruded to form a ring shaped bodies having an outside diameter of 6.5mm , a length of 6.5mm and a inner diameter of 2.5mm. Said ring shaped bodies were dried at a temperature from about 80̸°C to 10̸0̸°C for more than 2 hours to lower the water content of the bodies to below 25 percent and then calcined in a tunnel kiln, the temperature of which rose to 1480̸°C in 6 hours and remained constant for 6 hours. After the temperature of the tunnel kiln went down , pure alumina ring shaped bodies were obtained which had physical properties as follows:

| | |
|---|---|
| Crush strength kg/particle | 5.5 |
| Water absorption % | 68 |
| Bulk Density gm/cm³ | 0̸.55 |
| Specific surface area m²/g | 1.1 |
| pore volume ml/g | 0̸.56 |

| pore radius (micrometres) | distribution 〈% of total volume〉 |
|---|---|
| < 0̸.5 | 12.8 |
| 0̸.5-1 | 36.3 |
| 1 -5 | 23.0̸ |
| 5 -10̸ | 5.6 |
| 10̸ -30̸ | 5.8 |
| 30̸ -51 | 7.3 |
| >51 | 9.2 |

A catalyst was prepared by the following steps: 2.8 kilograms of silver nitrate was dissolved into 3 litres of deionised water, and 1.12 kilograms of ammonium oxalate was dissolved into 11 litres of deionised water at 50°C. The two solutions were mixed and white silver oxalate was precipitated. Then the precipitate was filtered and washed with distilled water until the washings contained no nitrate anions. The filter cake contained about 50 percent of silver and about 30 percent of water.

1.2 kilograms of ethyldiamine, 0.4 kilograms of diethanolamine, 1.5 kilograms of deionised water were added into a container made of stainless steel with a stirrer to get a mixed solution. The filter cake of silver oxalate was added into the mixed solution with stirring at below 40°C to dissolve all silver oxalate. Then 4.5 grams of barium nitrate, 9.5 grams of caesium sulphate and about 1 kilogram of deionised water were added into the mixed solution to form an impregnating solution which had about 22 percent of silver, 300 ppm of barium and 900 ppm of caesium.

3 kilograms of the carriers were placed into a container which can be evacuated to below 10mm Hg. The impregnation solution was added, and the carriers immersed for 30 minutes and then the extra solution dripped away. The impregnated alumina carriers were heated in an air flow at a temperature of about 550°C for a minute and the resulting silver catalyst contained 15 percent of silver, 200 ppm of barium and 600 ppm of caesium.

The catalysts were evaluated with a single tube reactor with an inner diameter of 21mm, a bed height of 7.07m and an effective volume of 2.45 litres. There is a jacket outside the reactor tube through which a heat conducting oil is passed to heat up the reactor or remove the heat of reaction. The conditions used for the evaluation were as follows:

| Constitution of the reaction gases: | |
|---|---|
| C₂H₄ | 20% mole |
| O₂ | 7% mole |
| CO₂ | 8% mole |
| N₂ | rest |
| Concentration of Ethylene Oxide in the effluent from the reactor | 1.3-1.4% mole |
| Reaction Pressure | 21 Kg/cm² |
| Space Velocity | 7000/h |
| Time-Space Yield | 180-200g Ethylene Oxide/l Cat.h |

and controlling the dichloroethane content of the reaction gas and making the selectivity of the catalyst the highest.

The selectivity of the catalysts prepared reaches 84.1% at a reaction temperature of 233°C.

### Example 2

Alumina ring shaped bodies, i.e. carriers, were prepared by means of the procedure similar to Example 1 except that 32 kilograms of graphite powder was substituted for petroleum coke and aluminium gel having about 10 percent of alumina was substituted for nitric acid for shaping. The alumina ring shaped bodies had physical properties as follows:

| | |
|---|---|
| Crush strength kg/particle | 6.5 |
| Water absorption rate % | 72.0 |
| Bulk density gm/cm² | 0.5 |
| Specific surface area m²/g | 1.1 |
| pore volume ml/g | 0̸.66 |

| pore radius (micrometres) | distribution 〈% of total volume〉 |
|---|---|
| < 0̸.5 | 7.3 |
| 0̸.5-1 | 27.0̸ |
| 1 -5 | 35.0̸ |
| 5 -10̸ | 6.2 |
| 10̸ -30̸ | 1.6 |
| 30̸ -51 | 15.0̸ |
| >51 | 7.9 |

A catalyst was prepared by means of the same procedure as in Example 1.

The catalyst was evaluated by a single tube in the same conditions as Example 1. The temperature of the reaction remains 235°C and the selectivity of the catalyst is 83.9%.

### Example 4-8

Alumina ring shaped bodies and silver catalysts were prepared by the same procedure as Example 1. A variety of catalysts containing different amounts of barium were prepared by changing the barium content in the impregation solution. The catalysts were evaluated in microreactor with an inner diameter of 4mm , a 8cm height of the catalyst bed , an effective volume of 1 ml and catalyst sizes of from 11 to 18 mesh.

The reaction gas mixtures were prepared in advance by mixing the concerned gases in a high pressure cylinder. The constitution of the reaction gas and the conditions for the evaluation were the same as Example 1. Dichloroethane, a restrainer in the reaction gas had a content of 0̸.4 ppm. The reaction temperature and the selectivity were measured. The results are shown in Table 1.

**Table 1**

| Examples | Ba in Impregnation solution ppm | Ag in catalyst % | Ba in catalyst ppm | Evaluation Temperature °C | Results | |
|---|---|---|---|---|---|---|
| | | | | | ΔEO % | S % |
| 4 | 0̸ | 15.6 | 0̸ | 222 | 1.39 | 82.1 |
| 5 | 30̸0̸ | 15.9 | 130̸ | 227 | 1.38 | 83.9 |
| 6 | 50̸0̸ | 15.4 | 350̸ | 224 | 1.38 | 83.2 |
| 7 | 70̸0̸ | 15.3 | 490̸ | 226 | 1.38 | 83.0̸ |
| 8 | 90̸0̸ | 14.9 | 610̸ | 226 | 1.38 | 82.7 |

### Comparative Example 3

Alumina bodies were prepared with the same prescription as Example 2 and by the same method as Example 1 except that the amount of added graphite was 2/5 of the amount used in Example 2. The physical properties of the alumina bodies prepared were as follows:

| | |
|---|---|
| Crush strength kg/particle | 9.3 |
| Water absorption rate % | 69.0̸ |
| Bulk Density gm/cm³ | 0̸.50̸ |
| specific surface area m²/g | 1.0̸6 |
| pore volume ml/g | 0̸.63 |

| pore radius (micrometres) | distribution 〈% of total volume〉 |
|---|---|
| < 0̸.5 | 8.5 |
| 0̸.5-1 | 12.5 |
| 1 -5 | 68.0̸ |
| 5 -10̸ | 7.5 |
| 10̸ -30̸ | 0̸.2 |
| 30̸ -51 | 1.2 |
| >51 | 1.8 |

A catalyst was prepared by the same method as Example 1.

The catalyst was evaluated in a single tube in the same conditions as Example 1. The reaction took place at a temperature of 237°C. The selectivity of the catalyst was 77.7 percent.

### Examples 9-11

Catalysts and alumina bodies were prepared in the same way as Example 1 except that the cesium content in impregnation solutions was changed and the evaluation of the catalysts was done in the same way as Example 4-8. the results are shown in Table 2.

**Table 2**

| Examples | Cs in Impregnation solution ppm | Ag in catalyst % | Cs in catalyst ppm | Evaluation Temperature °C | Results | |
|---|---|---|---|---|---|---|
| | | | | | ΔEO % | S % |
| 9 | 50̸0̸ | 12.3 | 280̸ | 231 | 1.39 | 83.3 |
| 10̸ | 70̸0̸ | 12.7 | 40̸0̸ | 236 | 1.39 | 84.4 |
| 11 | 90̸0̸ | 13.3 | 540̸ | 243 | 1.37 | 83.9 |

While the invention has been described in connection with what is presently considered to be the most practical and perferred embodiments , it is understood that the invention is not to be limited to the disclosed embodiments , but on the contrary , is intended to cover various modifications and equivalent arrangements included within the spirit and scope of the appended claims.

## Claims

1. A process for preparing a silver-containing catalyst for the production of ethylene oxide via ethylene oxidation, characterised by the steps of:-
(a) preparing a mixture of:-
(i) trihydrated α-alumina with a particle size of less than 50 mesh and boehmite alumina with a particle size of less than 200 mesh in proportions of from 1:1 to 9:1 based upon the weight of alumina,
(ii) a carbonaceous material having a particle size of from 20 to 200 mesh in an amount of from 10 to 40 percent of alumina by weight,
(iii)a fluxing agent,
(iv) a fluoride,
(v) a binder, and
(vi) water;
(b) extruding the mixture to form a shaped body;
(c) drying and calcining the shaped body to convert it to an alumina carrier having the following pore structure:-
| | |
|---|---|
| Specific surface area | 0.2-2m²/g |
| Pore volume | > 0.5 ml/g |
| Pore radius | Percent of the total volume |
|---|---|
| < 30 micrometres | 75-90 |
| > 30 micrometres | 25-10 |
provided that more than 10 percent of the total volume of the pore structure has a pore radius greater than 10 micrometres;
(d) impregnating said alumina carrier with a solution of a silver compound and, before, during or after that impregnation, a promoter; and
(e) reducing and activating said silver impregnated carrier.

2. A process according to Claim 1 characterised in that the carrier has a specific surface area of 0.8-1.3 m²/g and a pore volume of 0.5-0.7 ml/g.

3. A process according to Claim 1 or Claim 2 characterised in that the amount of carbonaceous material is 20 to 30 percent of alumina by weight.

4. A process according to any one of the preceding Claims characterised in that the trihydrated α-alumina has a low sodium content.

5. A process according to any one of the preceding Claims characterised in that the carbonaceous material is selected from the group consisting of petroleum coke, carbon powder, graphite, polyethylene, rosin and mixtures thereof.

6. A process according to any one of the preceding Claims characterised in that the amount of fluxing agent is from 1 to 7 percent of alumina by weight.

7. A process according to any one of the preceding Claims characterised in that the fluxing agent is selected from the group consisting of magnesium nitrate, magnesium oxide and feldspar.

8. A process according to any one of the preceding Claims characterised in that the amount of fluoride is from 0.5 to 5 percent of the weight of the alumina calculated on the fluoride anions.

9. A process according to any one of the preceding Claims characterised in that the fluoride is selected from the group consisting of aluminium fluoride, ammonium fluoride and hydrogen fluoride.

10. A process according to any one of the preceding Claims characterised in that the amount of binder is from 25 to 60 percent of the alumina by weight.

11. A process according to any one of the preceding Claims characterised in that the binder is selected from the group consisting of dilute nitric acid, alumina gel, propanoic acid, acetic acid and formic acid.

12. A process according to any one of the preceding Claims characterised in that the shaped body is a ring, cylinder, ball or perforated cylinder.

13. A process according to any one of the preceding Claims characterised in that the shaped body is dried to a water content of less than 30 percent prior to calcining.

14. A process according to Claim 13 characterised in that the shaped body is dried at a temperature in the range 80°C to 120°C for a period of 1 to 24 hours.

15. A process according to any one of the preceding Claims characterised in that the shaped body is calcined at a temperature in the range 1450°C to 1550°C for a period of 2 to 6 hours.

16. A process according to any one of the preceding Claims characterised in that the amount of silver content is from 1 to 25 percent of the weight of the catalyst and, more preferably, from 5 to 20 percent of the weight of the catalyst.

17. A process according to any one of the preceding Claims characterised in that the alumina carrier is impregnated by a solution of the silver compound and the promoter.

18. A process according to any one of Claims 1 to 16 characterised in that the impregnation with the promoter is carried out after the reduction of the silver impregnated carrier.

19. A process according to any one of the preceding Claims characterized in that the promoter is at least one alkali metal and/or alkaline earth metal.

20. A process according to any one of the preceding Claims characterized in that the promoter is an alkali metal in an amount (calculated on metal) from 20, preferably 40, to 1000 parts per million parts of catalyst by weight.

21. A process according to Claim 19 or Claim 20 characterised in that the alkali metal is selected from the group consisting of potassium, rubidium and caesium.

22. A process according to any one of Claims 1 to 19 characterised in that the promoter is an alkaline earth metal in an amount (calculated on metal) less than 1000 parts per million parts of catalyst by weight.

23. A process according to Claim 22 characterised in that the alkaline earth metal is barium.

24. A silver catalyst made by the process according to any one of the preceding Claims.

25. A silver catalyst according to Claim 24 characterised in that the ratio of the percent of the total volume of the pore structure having a pore radius not greater than 10 micrometres to that having a pore radius not less than 10 micrometres is approximately 3:1.

26. Use of the silver catalyst according to Claim 24 or Claim 25 for the production of ethylene via ethylene oxidation.

## Patentansprüche

1. Verfahren zur Herstellung eines silberhaltigen Katalysators zur Produktion von Ethylenoxid über Oxidation von Ethylen, gekennzeichnet durch die Stufen, in denen
(a) eine Mischung aus
(i) trihydratisiertem α-Aluminiumoxid mit einer Teilchengröße von weniger als 50 mesh und Böhmit-Aluminiumoxid mit einer Teilchengröße von weniger als 200 mesh in Proportionen von 1:1 bis 9:1, bezogen auf das Gewicht des Aluminiumoxids,
(ii) einem kohlenstoffhaltigen oder kohlenstoffartigen Material mit einer Teilchengröße von 20 bis 200 mesh in einer Menge von 10 bis 40 Gew.% des Aluminiumoxids;
(iii) einem Flußmittel;
(iv) einem Fluorid;
(v) einem Bindemittel und
(vi) Wasser hergestellt wird;
(b) die Mischung unter Bildung eines geformten Körpers extrudiert wird;
(c) der geformte Körper getrocknet und calciniert wird, um ihn in einen Aluminiumoxidträger mit der folgenden Porenstruktur zu überführen:
| | |
|---|---|
| spezifische Oberfläche | 0,2 bis 2 m²/g |
| Porenvolumen | > 0,5 ml/g |
| Porenradius | Prozent des Gesamtvolumens |
|---|---|
| < 30 »m | 75 bis 90 |
| > 30 »m | 25 bis 10 |
mit der Maßgabe, daß mehr als 10 % des Gesamtvolumens der Porenstruktur einen Porenradius größer als 10 »m aufweist;
(d) der Aluminiumoxidträger mit einer Lösung einer Silberverbindung imprägniert wird und vor, während oder nach dieser Impägnierung mit einem Promoter imprägniert wird, und
(e) der mit Silber imprägnierte Träger reduziert und aktiviert wird.

2. Verfahren nach Anspruch 1, dadurch gekennzeichnet, daß der Träger eine spezifische Oberfläche von 0,8 bis 1,3 m²/g und ein Porenvolumen von 0,5 bis 0,7 ml/g aufweist.

3. Verfahren nach Anspruch 1 oder Anspruch 2, dadurch gekennzeichnet, daß die Menge an kohlenstoffartigem oder kohlenstoffhaltigem Material 20 bis 30 Gew.% des Aluminiumoxids beträgt.

4. Verfahren nach einem der vorhergehenden Ansprüche, dadurch gekennzeichnet, daß das trihydratisierte α-Aluminiumoxid einen niedrigen Natriumgehalt hat.

5. Verfahren nach einem der vorhergehenden Ansprüche, dadurch gekennzeichnet, daß das kohlenstoffhaltige oder kohlenstoffartige Material ausgewählt ist aus der Gruppe bestehend aus Erdölkoks, Kohlenstoffpulver, Graphit, Polyethylen, Kolophonium und Mischungen derselben.

6. Verfahren nach einem der vorhergehenden Ansprüche, dadurch gekennzeichnet, daß die Menge an Flußmittel 1 bis 7 Gew.% des Aluminiumoxids beträgt.

7. Verfahren nach einem der vorhergehenden Ansprüche, dadurch gekennzeichnet, daß das Flußmittel ausgewählt ist aus der Gruppe bestehend aus Magnesiumnitrat, Magnesiumoxid und Feldspat.

8. Verfahren nach einem der vorhergehenden Ansprüche, dadurch gekennzeichnet, daß die Menge an Fluorid 0,5 bis 5 Gew.% des Aluminiumoxids beträgt, berechnet als Fluoridanionen.

9. Verfahren nach einem der vorhergehenden Ansprüche, dadurch gekennzeichnet, daß das Fluorid ausgewählt ist aus der Gruppe bestehend aus Aluminiumfluorid, Ammoniumfluorid und Wasserstofffluorid.

10. Verfahren nach einem der vorhergehenden Ansprüche, dadurch gekennzeichnet, daß die Menge an Bindemittel 25 bis 60 Gew.% des Aluminiumoxids beträgt.

11. Verfahren nach einem der vorhergehenden Ansprüche, dadurch gekennzeichnet, daß das Bindemittel ausgewählt ist aus der Gruppe bestehend aus verdünnter Salpetersäure, Aluminiumoxidgel, Propionsäure, Essigsäure und Ameisensäure.

12. Verfahren nach einem der vorhergehenden Ansprüche, dadurch gekennzeichnet, daß der geformte Körper ein Ring, ein Zylinder, eine Kugel oder ein perforierter Zylinder ist.

13. Verfahren nach einem der vorhergehenden Ansprüche, dadurch gekennzeichnet, daß der geformte Körper vor dem Calcinieren auf einen Wassergehalt von weniger als 30 % getrocknet wird.

14. Verfahren nach Anspruch 13, dadurch gekennzeichnet, daß der geformte Körper für eine Zeitdauer von 1 bis 24 Stunden bei einer Temperatur im Bereich von 80°C bis 120°C getrocknet wird.

15. Verfahren nach einem der vorhergehenden Ansprüche, dadurch gekennzeichnet, daß der geformte Körper für eine Zeitdauer von 2 bis 6 Stunden bei einer Temperatur im Bereich von 1450°C bis 1550°C calciniert wird.

16. Verfahren nach einem der vorhergehenden Ansprüche, dadurch gekennzeichnet, daß die Menge des Silbergehalts 1 bis 25 % des Katalysatorgewichts und insbesondere 5 bis 20 % des Katalysatorgewichts beträgt.

17. Verfahren nach einem der vorhergehenden Ansprüche, dadurch gekennzeichnet, daß der Aluminiumoxidträger durch eine Lösung der Silberverbindung und des Promoters imprägniert wird.

18. Verfahren nach einem der Ansprüche 1 bis 16, dadurch gekennzeichnet, daß die Imprägnierung mit dem Promoter nach der Reduktion des mit Silber imprägnierten Trägers durchgeführt wird.

19. Verfahren nach einem der vorhergehenden Ansprüche, dadurch gekennzeichnet, daß der Promoter mindestens ein Alkalimetall und/oder Erdalkalimetall ist.

20. Verfahren nach einem der vorhergehenden Ansprüche, dadurch gekennzeichnet, daß der Promoter ein Alkalimetall in einer Menge (berechnet als Metall) von 20, vorzugsweise 40, bis 1000 ppm, bezogen auf das Gewicht des Katalysators, ist.

21. Verfahren nach Anspruch 19 oder Anspruch 20, dadurch gekennzeichnet, daß das Alkalimetall ausgewählt ist aus der Gruppe bestehend aus Kalium, Rubidium und Cäsium.

22. Verfahren nach einem der Ansprüche 1 bis 19, dadurch gekennzeichnet, daß der Promoter ein Erdalkalimetall in einer Menge (berechnet als Metall) von weniger als 1000 ppm, bezogen auf das Gewicht des Katalysators, ist.

23. Verfahren nach Anspruch 22, dadurch gekennzeichnet, daß das Erdalkalimetall Barium ist.

24. Silberkatalysator, der nach dem Verfahren gemäß einem der vorhergehenden Ansprüche hergestellt worden ist.

25. Silberkatalysator nach Anspruch 24, dadurch gekennzeichnet, daß das Verhältnis der Prozent des Gesamtvolumens der Porenstruktur mit einem Porenradius von nicht mehr als 10 »m zu den Prozent mit einem Porenradius von nicht weniger als 10 »m ungefähr 3:1 beträgt.

26. Verwendung des Silberkatalysators gemäß Anspruch 24 oder Anspruch 25 zur Herstellung von Ethylenoxid über Oxidation von Ethylen.

## Revendications

1. Procédé de préparation d'un catalyseur contenant de l'argent pour la production d'oxyde d'éthylène par oxydation d'éthylène, caractérisé par les stades suivants :
(a) on prépare un mélange :
(i) de α-alumine trihydratée d'un calibre particulaire inférieur à 50 mesh et d'alumine de boehmite d'un calibre particulaire inférieur à 200 mesh dans des proportions de 1:1 à 9:1 sur base du poids de l'alumine,
(ii) d'un matériau carboné d'un calibre particulaire de 20 à 200 mesh en quantité de 10 à 40 % de l'alumine en poids,
(iii) d'un fondant,
(iv) d'un fluorure,
(v) d'un liant, et
(vi) d'eau;
(b) on extrude le mélange pour former un corps moulé;
(c) on sèche et on calcine le corps moulé pour le convertir en un support d'alumine ayant la structure poreuse suivante:
| | |
|---|---|
| Surface spécifique | 0,2-2 m²/g |
| Volume de pores | > 0,5 ml/g |
| Rayon des pores | Pourcentage du volume total |
|---|---|
| < 30 micromètres | 75-90 |
| > 30 micromètres | 25-10 |
pourvu qu'une quantité de plus de 10 % du volume total de la structure poreuse ait un rayon de pores supérieur à 10 micromètres;
(d) on imprègne ledit support d'alumine d'une solution d'un composé d'argent et, avant, pendant ou après cette imprégnation, d'un promoteur; et
(e) on réduit et on active ledit support imprégné d'argent.

2. Procédé selon la revendication 1, caractérisé en ce que le support a une surface spécifique de 0,8 à 1,3 m²/g et un volume de pores de 0,5 à 0,7 ml/g.

3. Procédé selon la revendication 1 ou 2, caractérisé en ce que la quantité de matière carbonée est de 20 à 30 % de l'alumine en poids.

4. Procédé selon l'une quelconque des revendications précédentes, caractérisé en ce que la α-alumine trihydratée a une teneur en sodium faible.

5. Procédé selon l'une quelconque des revendications précédentes, caractérisé en ce que la matière carbonée est choisie dans le groupe formé du coke de pétrole, de la poudre de carbone, du graphite, du polyoxyéthylène, de la colophane et de leurs mélanges.

6. Procédé selon l'une quelconque des revendications précédentes, caractérisé en ce que la quantité de fondant est de 1 à 7 % de l'alumine en poids.

7. Procédé selon l'une quelconque des revendications précédentes, caractérisé en ce que le fondant est choisi dans le groupe formé du nitrate de magnésium, de l'oxyde de magnésium et du feldspath.

8. Procédé selon l'une quelconque des revendications précédentes, caractérisé en ce que la quantité de fluorure est de 0,5 à 5 % en poids de l'alumine, calculée sur base des anions de fluorure.

9. Procédé selon l'une quelconque des revendications précédentes, caractérisé en ce que le fluorure est choisi dans le groupe formé du fluorure d'aluminium, de fluorure d'ammonium et du fluorure d'hydrogène.

10. Procédé selon l'une quelconque des revendications précédentes, caractérisé en ce que la quantité de liant est de 25 à 60 % de l'alumine en poids.

11. Procédé selon l'une quelconque des revendications précédentes, caractérisé en ce que le liant est choisi dans le groupe formé de l'acide nitrique dilué, du gel d'alumine, de l'acide propanoïque, de l'acide acétique et de l'acide formique.

12. Procédé selon l'une quelconque des revendications précédentes, caractérisé en ce que le corps moulé est un anneau, un cylindre, une bille ou un cylindre perforé.

13. Procédé selon l'une quelconque des revendications précédentes, caractérisé en ce que le corps moulé est séché jusqu'à ce qu'il ait une teneur en eau inférieure à 30 % avant calcination.

14. Procédé selon la revendication 13, caractérisé en ce que le corps moulé est séché à une température de la plage de 80 à 120°C pendant une période de 1 à 24 heures.

15. Procédé selon l'une quelconque des revendications précédentes, caractérisé en ce que le corps moulé est calciné à une température dans la plage de 1450 à 1550°C pendant une période de 2 à 6 heures.

16. Procédé selon l'une quelconque des revendications précédentes, caractérisé en ce que la quantité d'argent est de 1 à 25 % en poids du catalyseur, mieux encore, de 5 à 20 % du poids du catalyseur.

17. Procédé selon l'une quelconque des revendications précédentes, caractérisé en ce que le support d'alumine est imprégné d'une solution du composé d'argent et du promoteur.

18. Procédé selon l'une quelconque des revendications 1 à 16, caractérisé en ce que l'imprégnation par le promoteur est réalisée après la réduction du support imprégné d'argent.

19. Procédé selon l'une quelconque des revendications précédentes, caractérisé en ce que le promoteur est au moins un métal alcalin et/ou un métal alcalino-terreux.

20. Procédé selon l'une quelconque des revendications précédentes, caractérisé en ce que le promoteur est un métal alcalin en quantité (calculée sur base du métal) de 20, de préférence 40, à 1000 parties par million de catalyseur en poids.

21. Procédé selon la revendication 19 ou 20, caractérisé en ce que le métal alcalin est choisi dans le groupe formé du potassium, du rubidium et du césium.

22. Procédé selon l'une quelconque des revendications 1 à 19, caractérisé en ce que le promoteur est un métal alcalino-terreux en quantité (calculée sur base du métal) inférieure à 1000 parties par million de catalyseur en poids.

23. Procédé selon la revendication 22, caractérisé en ce que le métal alcalino-terreux est le baryum.

24. Catalyseur d'argent réalisé par le procédé selon l'une quelconque des revendications précédentes.

25. Catalyseur d'argent selon la revendication 24, caractérisé en ce que le rapport du pourcentage du volume total de la structure poreuse ayant un rayon de pores non supérieur à 10 micromètres à celui ayant un rayon de pores non inférieur à 10 micromètres est d'environ 3:1.

26. Utilisation du catalyseur d'argent selon la revendication 24 ou 25 pour la production d'oxyde d'éthylène par oxydation d'éthylène.
